Europäisches Patentamt

European Patent Office

Office européen des brevets

(19)

(11) Publication number: **0 440 303 A1**

# EUROPEAN PATENT APPLICATION

(21) Application number: **91200189.8**

(22) Date of filing: **30.01.91**

(51) Int. Cl.⁵: **C12N 9/48**, C12N 15/57,
A23C 19/032, A23C 13/16,
A23L 1/31, C12P 21/08,
G01N 33/68

(30) Priority: **31.01.90 NL 9000241**

(43) Date of publication of application:
**07.08.91 Bulletin 91/32**

(84) Designated Contracting States:
**AT BE CH DE DK ES FR GB IT LI NL SE**

(71) Applicant: **PROBICOM RESEARCH B.V.
Snekerweg 15
NL-8701 XB Bolsward(NL)**

(72) Inventor: **Bosman, Boukje Wendelina
Hoornsediep 24
NL-9725 HK Groningen(NL)**
Inventor: **Tan, Paris Som Tjwan
Vinkenstraat 122A
NL-9713 TM Groningen(NL)**
Inventor: **Konings, Wilhelmus Nicolaas
Rijksstraatweg 236
NL-9752 CJ Haren(NL)**

(74) Representative: **Smulders, Theodorus A.H.J.,
Ir. et al
Vereenigde Octrooibureaux Nieuwe Parklaan
107
NL-2587 BP 's-Gravenhage(NL)**

(54) **Tripeptidase.**

(57) The present invention relates to a tripeptidase of lactic acid bacteria in isolated form as well as to uses thereof, e.g., in the preparation of foods such as cheese, whippable products and meat products, or for preparing antibodies against the tripeptidase. The invention also relates to recombinant genetic information coding for the tripeptidase and transformed organisms expressing the tripeptidase. The tripeptidase can be isolated from Lactococcus lactis subsp. cremoris Wg2 and specifically separates the N-terminal amino acid from tripeptides. The hydrolysis activity has a temperature optimum at about 55°C and a pH optimum at about 7.5. The molecular weight is about 105 kD and the isoelectric point about 3.7. The tripeptidase is sensitive to EDTA, dithiothreitol and β-mercaptoethanol.

EP 0 440 303 A1

# TRIPEPTIDASE

This invention relates to a tripeptidase of lactic acid bacteria in isolated form, i.e. in a substantially pure condition in relation to the condition in which it is present in lactic acid bacteria in vivo.

Furthermore, the invention relates to transformed organisms, in particular microorganisms (bacteria, but also fungi and yeasts), capable of expression of the relevant tripeptidase (i.e. capable of forming the tripeptidase); to nucleotide sequences (in particular DNA) coding for the tripeptidase; to a recombinant polynucleotide, more in particular recombinant DNA comprising the genetic information coding for the tripeptidase, as well as to the use of such a recombinant polynucleotide as a transformation marker; to a process for preparing the tripeptidase by isolation from a crude cell-free extract of an organism in which the tripeptidase is expressed, e.g., from lactic acid bacteria; to the use of the tripeptidase for preparing polyclonal and monoclonal antibodies against the tripeptidase (i.e. having affinity and/or specificity for the tripeptidase), as well as to such antibodies per se and to their use for detecting, removing and/or isolating the tripeptidase; to a process for preparing foods, such as cheese and other dairy products, meat products and protein hydrolysates, in which the tripeptidase is used, as well as to the foods thus obtained.

Lactic acid bacteria, in particular lactic acid Lactococci such as Lactococcus lactis subsp. cremoris, lactis and diacetylactis, as well as other lactic acid bacteria such as Leuconostoc species are largely used in the food industry, in particular in the dairy industry for preparing fermented milk products such as cheese. By producing lactic acid from lactose the food product is acidified, thus protecting the food product against decay. Moreover, the bacteria are involved in the development of flavour and taste of the food product. In this connection an important process is the breakdown of proteins.

For growth and for lactic acid production the bacteria require a nutrient medium providing essential growth factors such as vitamins and amino acids. Milk is a suitable nutrient medium and enables growth to high cell densities. For this purpose, however, the content of free amino acids is insufficient in milk, so that as far as their need for amino acids is concerned the bacteria depend on their proteolytic system for the breakdown of milk proteins, in particular casein. The proteolytic system of lactic acid bacteria also remains active after terminated growth, e.g., in the cheese ripening phase in which the breakdown of casein to peptides and then amino acids contribute to the flavour and taste development of the cheese.

The proteolytic system of lactococci comprises different proteinases and different peptidases. The proteinases are localized in the cell wall of the bacteria, whereas the peptidases may be present in the medium, in the cell wall, and within the cell.

Of lactococci some peptidases have meanwhile been isolated and characterized.

Law, J.Appl.Bacteriol. 46 (1979) 455-463, described the presence of di-, tri- and aminopeptidase activities in L.lactis subsp. cremoris NCDO 1196 and 2016 and L.lactis subsp.lactis NCDO 763. Different aminopeptidase activities were purified. Geis et al., Appl.Microbiol. Biotechnol. 23 (1985) 79-84, isolated and characterized a cell wall-associated aminopeptidase of L.lactis subsp. cremoris AC1. Exterkate et al., Appl.Environ. Microbiol. 53 (1987) 577-583, purified a membrane-bound aminopeptidase of L.lactis subsp. cremoris HP. Of L.lactis subsp. cremoris AM2, Neviani et al., Appl.Environ. Microbiol. 55 (1989) 2308-2314 purified an intracellular aminopeptidase (API). Desmazeaud et al., Milchwissenschaft 34 (1979) 606-610, isolated an intracellular aminopeptidase of L.lactis subsp. diacetylactis CNRZ 267. And Tan et al., Appl. Environ. Microbiol. 56 (1990) 526-532, purified an aminopeptidase of L.lactis subsp. cremoris Wg2. Desmazeaud et al., Ann.Biol. Anim. Bioch. Biophys. 17 (1977) 723-736 and Milchwissenschaft 34 (1979) 606-610, purified from L.lactis subsp. diacetylactis CNRZ 267 respectively a dipeptidase and an intracellular tripeptidase (APII). The peptidases of L.lactis subsp. cremoris H61 were examined extensively. Yan et al., Eur. J. Biochem. 163 (1987) 259-265 and Appl. Environ. Microbiol. 53 (1987) 2296-2302, purified two endopeptidases; Hwang et al., Agric.Biol.Chem. 45 (1981) 159-165, isolated a dipeptidase, and Kaminogawa et al., Agric.Biol.Chem. 48 (1984) 3035-3040, isolated and characterized a prolidase. Of L.lactis subsp. cremoris p8-2-47, Kiefer-Partsch et al., Appl.Microbiol. Biotechnol. 31 (1989) 75-78, isolated a cell wall-bound X-prolyl dipeptidyl peptidase. From L.lactis subsp. lactis NCDO 763 Zevaco et al., Appl.Bacteriol. 68 (1990) 357-366 isolated an intracellular x-prolyl dipeptidyl peptidase. And Van Boven et al., Appl. Environ. Microbiol. 54 (1988) 43-49, purified a dipeptidase from L.lactis subsp. cremoris Wg2.

In the Dutch cheese industry L.lactis subsp. cremoris is the most important lactic acid bacterial species. A good insight into the proteolytic system of lactococci such as L.lactis subsp. cremoris may contribute to an improvement of the starter cultures and to a better control of the ripening process, e.g., thus realizing accelerated cheese ripening or preventing development of a bitter taste (formation of bitter peptides). Much basic information is given in the thesis by R. Otto entitled "An ecophysiological study of starter streptococci", State University of Groningen (1981) and in the thesis by J. Hugenholtz entitled "Population dynamics of mixed starter cultures", State University of Groningen (1986). The latter thesis describes a study into the proteinases of L.lactis subsp. cremoris Wg2, and the thesis by A. van Boven entitled "The uptake and hydrolysis of peptides by S.cremoris" describes a further study into the proteolytic system of L.lactis subsp. cremoris Wg2.

The present invention is based on a further study into the peptidases of L.lactis subsp. cremoris Wg2, which comprises isolating and characterizing a specific tripeptidase which had not been described previously. The tripeptidase was purified from a cell-free extract of L.lactis subsp. cremoris Wg2 by DEAE-Sephacel column chromatography followed by phenyl-Sepharose chromatography and gel filtration over a Sephadex column and a "high performance liquid chromatography" (HPLC) gel filtration column. The tripeptidase shows hydrolysis activity with respect to several tripeptides and is, in view of its sensitivity to the metal chelating compound EDTA, a metalloenzyme showing a pH optimum at about 7.5 and a temperature optimum at about $55^\circ$ C. In sodium dodecyl sulphate polyacrylamide gel electrophoresis (SDS-PAGE) of the purified enzyme one single protein band is found having a molecular weight of about 105 kD. The enzyme is subjected to strong inhibition of compounds such as $\beta$-mercaptoethanol and dithiotreitol, which are capable of reducing disulphide linkages, and is not inhibited by the sulfhydryl group reagents p-chloromercuribenzoate (pCMB),

p-chloromercuribenzene sulphonate (pCMBS) and

O-(3-hydroxymercuri-2-methoxypropyl) carbamylphenoxyacetate (mersalyl). Kinetic studies show that the peptidase leucyl-leucyl-leucine (leu₃) hydrolyses at a $K_m$ of 0.15 mM and a $V_{max}$ of 151 $\mu$mol/min. mg protein. This value corresponds to a turnover number of about 264 sec⁻¹. The pl (isoelectric point) of the enzyme is relatively low (about 3.7).

The novel tripeptidase according to the invention differs from an intracellular tripeptidase of L.lactis subsp. cremoris NCDO 1196 and an intracellular tripeptidase, a superficial tripeptidase and a cell wall tripeptidase of L.lactis subsp. lactis NCDO 763 described by Law et al. These enzymes have other molecular weights and temperature optima. None of both intracellular enzymes is inhibited by $\beta$-mercaptoethanol, and the superficial and cell wall enzymes of L.lactis subsp. lactis NCDO 763 can be reactivated after inhibition by EDTA with $Mg^{2+}$. As for pH optima and inhibition by EDTA, however, these enzymes show similarities. Both intracellular enzymes are likewise reactivated by $Zn^{2+}$, $Mn^{2+}$ and $Co^{2+}$ after EDTA treatment, and the superficial and cell wall tripeptidases of L.lactis subsp. lactis NCDO 763 are also inhibited by $\beta$-mercaptoethanol.

The novel tripeptidase also differs from the intracellular tripeptidase (APII) of L.lactis subsp. diacetylactis CNRZ 267 as described by Desmazeaud et al. The two tripeptidase enzymes differ as regards molecular weight, temperature optima and inhibition by pCMB. The enzymes, however, show similarities in pH optima, inhibition by EDTA and reactivation by $Mn^{2+}$ and $Co^{2+}$ after EDTA treatment.

The novel tripeptidase could be used to prevent development of an undesirable bitter taste during cheese ripening. Actually, it has been found that after casein breakdown some L.lactis subsp. cremoris strains produce different grades of bitterness, apparently as a result of the accumulation of peptides having a high content of hydrophobic amino acids which are only slowly broken down by the peptidases of the bacteria present in the starter. The use of the present tripeptidase could mitigate this problem.

Furthermore, the tripeptidase may be used for other purposes, such as clarification of beer and beverages, and it may of course also be used in DNA technology in a wide sense.

The invention is first embodied in the tripeptidase itself, i.e. a tripeptidase of lactic acid bacteria in isolated form, characterized by a molecular weight of 105 kD ± 5 kD; an isoelectric point of 3.7 ± 0.4; a substrate range which comprises only tripeptides, with the exception of that with proline as the second amino acid, and which does not comprise dipeptides, nor oligopeptides such as ala₄, ala₅, ala₆, nor polypeptides; a hydrolysis activity with a temperature optimum at $55^\circ$ C ± $5^\circ$ C and a pH optimum at 7.5 ± 0.5; and sensitivity to both the metal chelating agent EDTA and to the reducing agents dithiothreitol and $\beta$-mercaptoethanol.

The tripeptidase is further characterized by the following amino acid sequence at the N-terminal end:

met-lys-tyr-glu-lys-leu-leu-pro-arg-phe-leu-glu-tyr-val-lys-val-asn-thr-arg-ser-asp-glu-asn-ser-thr.

Although the tripeptidase which has actually been isolated and characterized according to the experimental part herein originates from one specific organism, namely a proteinase negative variant of L.lactis subsp. cremoris Wg2, the invention also comprises the corresponding tripeptidases of other lactic acid bacteria, since these have corresponding properties and can be isolated and purified from the bacteria in a corresponding manner. By "corresponding tripeptidases" is not meant that the peptidases are exactly equal, i.e. have the same amino acid sequence, but is rather meant that they satisfy the above characterization of the tripeptidase.

A more specific embodiment of the invention is concerned with the tripeptidase originating from lactococci, in particular L.lactis subsp. cremoris, most preferably L.lactis subsp. cremoris Wg2.

By the words "in isolated form" and "originating from" is not meant to limit the invention to tripeptidases that have been isolated from their natural environment. In fact, the invention creates the possibility of tracing the tripeptidase gene and then realizing production of the tripeptidase in transformed cells or organisms by means of recombinant DNA technology. For this purpose, e.g., the amino acid sequence of the tripeptidase, or of a part thereof, could be determined. On the basis thereof, corresponding DNA sequences may be derived by means of the genetic code, after which suitable DNA probes may be synthesized with which tripeptidase messenger RNA (mRNA) of the cells can be detected and isolated. This mRNA may be used by known per se techniques for preparing copy DNA (cDNA) using enzymes such as reverse transcriptase and DNA polymerase. This cDNA may be cloned by means of suitable vectors (mostly plasmids) and expressed in suitable host cells, from which the tripeptidase produced can be easily recovered.

On the basis of such genetic work, there can also be constructed a marker plasmid which comprises the tripeptidase gene fused with another desired gene. By means of transformation of such a plasmid into a tripeptidaseless host an expression of the fusion product can be obtained, for which it can be easily screened by means of, e.g., the specific tripeptidase substrate leu₃ or by means of poly- or monoclonal antibodies against the tripeptidase. Such a marker plasmid may be of great significance to the food industry because the plasmid is derived from a bacterial species generally regarded as safe (i.e. a GRAS organism).

The tripeptidase according to the invention may be obtained by isolation thereof from a cell mass, a culture medium or a separation product of lactic acid bacteria, in which the tripeptidase is naturally expressed, or from a cell mass, a culture medium or a separation product of an organism which, by means of genetic engineering, has acquired the ability of expressing the tripeptidase.

The invention is further also embodied in organisms, in particular microorganisms, which, by means of genetic engineering, have acquired either the ability foreign to them of expressing a tripeptidase according to the invention or the ability of a stronger expression of a tripeptidase according to the invention than they naturally possess.

The invention is also embodied in a recombinant polynucleotide, in particular recombinant DNA, comprising the genetic information coding for a tripeptidase according to the invention. This recombinant polynucleotide may only consist of the gene coding for the tripeptidase or may also comprise regulation sequences such as, e.g., a suitable transcription promoter and/or a vector part.

The invention further comprises the use of such a recombinant polynucleotide as a transformation marker in which a successful transformation of a tripeptidase-negative organism is concluded from expression of the tripeptidase for which the polynucleotide comprises the genetic information, which expression is detected by means of a suitable substrate of the tripeptidase or by means of polyclonal or monoclonal antibodies having affinity and/or specificity for the tripeptidase. The invention also includes the use as a transformation marker in the form of a plasmid containing the tripeptidase as a gene, which gene is inactivated by introducing another desired gene.

The invention further comprises a process for preparing a tripeptidase according to the invention, which process is characterized in that a cell-free extract of an organism, in particular a microorganism, in which the tripeptidase is expressed, is subjected to one or more ion exchange column chromatography and gel filtration steps in which of each eluate collected in fractions one or more of the fractions having hydrolysis activity with respect to leu₃ and/or leu-gly-gly are selected.

Moreover, the invention creates the possibility to produce antibodies against the tripeptidase and then to use them to remove or isolate the tripeptidase from its natural environment or from the above transformed cells or organisms.

The invention is therefore also embodied in the use of the novel tripeptidase for preparing polyclonal or monoclonal antibodies having affinity and/or specificity for the tripeptidase, in these polyclonal and monoclonal antibodies themselves, and in their use for detecting the tripeptidase in, or removing or isolating it from a mixture containing the tripeptidase.

Of course, the invention is further embodied in a process for preparing foods such as cheese, whippable products and meat products, using a tripeptidase and/or an organism according to the invention. Here specific hydrolysis properties of the tripeptidase can be used, e.g., to inhibit the development of a bitter flavour or to realize accelerated cheese ripening. The process may also have the advantage of lower production costs, e.g., because less starter is required. The foods obtained by this process are also comprised by the invention, in particular if they are distinguished from well-known products by a higher quality (better taste or, more in general, better organoleptic properties).

The invention will hereinafter be explained by means of the experimental part.

## Materials and methods

### Organism and preparation of a cell-free extract

In the experiments a proteinase-negative variant of L.lactis subsp. cremoris Wg2 was used. The organism was stored by way of routine in 10% (wt./vol.) sterile reconstituted skim milk containing 0.1% (wt./vol.) tryptone, at a temperature of -20°C. For culturing L.lactis subsp. cremoris Wg2 a 1% (wt./vol.) lactosecontaining MRS medium (De Man et al., J.Appl.Bacteriol. 23, 1960, 130-135) was used, the growth occurring at a controlled pH of 6.3 in a 5 litre fermenter. Cells were harvested in the exponential growth phase at $A_{660nm}$ of 0.8, washed (twice) in 10 mM $K_2HPO_4$-$KH_2PO_4$ (KPi; pH 7.0) and suspended in 30 ml of this buffer. Cells were broken by sonication (Soniprep 150; MSE Scientific Instruments, Crawley, England) for 480 sec. (8x 15 sec. sonification and 45 sec. rest) at 10 μm and 4°C under a constant nitrogen stream. The broken cells were centrifuged for 30 min. at 20,700 g and 0°C, and the resulting supernatant (i.e. cell-free extract) was used for enzyme isolation.

### Enzyme determinations

Peptidase activity was determined by measuring the release of leucine from the tripeptide leucyl-leucyl-leucine ($leu_3$) (2 mM in 20 mM 4-(2-hydroxyethyl)-1-piperazine-ethane-sulphonic acid [HEPES], pH 7.0). Enzyme and substrate were incubated for 10 min. at 30°C, and the release of leucine was measured by the modified cadmium-ninhydrin method as described by Doi et al., Anal.Biochem. 118, 1981, 173-184. Hydrolysis of peptides was detected by thin-layer chromatography (TLC). Peptidase activity was determined as follows: the reaction mixture containing 2 mM substrate in 20 mM HEPES (pH 7.0) and a suitable amount of enzyme was incubated for 60 min. at 30°C. The reaction mixture (10μl) was then placed on a precoated silica gel 60 plate having a layer thickness of 0.25 cm (Merck, Darmstadt, Germany). A 75% (wt./vol.) phenol/water mixture of a 4/l/l (vol./vol./vol.) mixture of n-butanol, acetic acid and water was used as mobile phase. Peptides and amino acids were detected after spraying the silica gels with 0.1% (wt./vol.) ninhydrin in 99% ethanol and 5 min incubation at 80°C.

### DEAE-Sephacel column chromatography

A DEAE-Sephacel column (Pharmacia LKB Biotechnology, Uppsala, Sweden; 1.6 x 20 cm) was equilibrated with 10 mM KPi (pH 7.0) with 0.12 M NaCl. The cell-free extract was diluted with distilled water to obtain a lower ion strength than the buffer used for equilibrating the DEAE-Sephacel column and was then placed on the column. The elution was carried out with a linear gradient of 0.12 M to 0.40 M NaCl in 10 mM KPi (pH 7.0). Fractions were tested for $leu_3$ hydrolysis activity. The active fractions were placed on a Phenyl-Sepharose column.

### Phenyl Sepharose column chromatography

A Phenyl-Sepharose (CL4B) column (Pharmacia LKB Biotechnology, Uppsala, Sweden; 1.8 x 8.5 cm) was equilibrated with 10 mM KPi (pH 7.0) containing 4 M NaCl. NaCl was added to the enzyme fraction up to 4 M NaCl, after which the enzyme solution was placed on the column. The column was eluted with a reverse gradient of 4 M NaCl to 0 M NaCl in 10 mM KPi (pH 7.0). Fractions showing leucyl-glycyl-glycine (leu-gly-gly) hydrolysis were collected and concentrated 65-fold in an Amicon filtration unit with a YM-10 membrane (Amicon Corp., Lexington, USA). The concentrate was placed on a Sephadex G-100 SF gel filtration column.

### Gel filtration chromatography

Gel filtration was carried out with a Sephadex G-100 SF (Pharmacia LKB Biotechnology) column (1.6 x 199 cm).

The column was eluted with 50 mM KPi (pH 7.0) containing 0.1 M NaCl. The enzyme sample was supplemented with glycerol up to 33% (vol/vol) before it was placed on the column. Fractions with $leu_3$ hydrolysis activity were collected and concentrated 6-fold in the manner as described above.

### High Performance liquid gel filtration chromatography

Enzyme fractions of 100 $\mu$l were injected onto an Ultrapac TSK G3000 SW gel filtration column (7.5 x 600 mm) (Pharmacia LKB Biotechnology). The column was eluted with 20 mM KPi (pH 7.0) containing 60 mM NaCl (HPLC: Waters 600 Multisolvent Delivery System with a U6K universal liquid chromatograph injector). Protein was measured at $A_{214\ nm}$ with a Lambda Max model 481 LC spectrophotometer (Millipore, Waters Associates, Milford, Mass.) and peak fractions were tested for $leu_3$ hydrolysis.

## SDS-PAGE

Sodium dodecyl sulphate (SDS) polyacrylamide gel electrophoresis (PAGE) was carried out as described by Laemmli and Faure, J.Mol.Biol. 80, 1973, 575-599 with 10% acrylamide-containing gels. The protein samples were diluted 4 : 1 with sample buffer (50 mM Tris-HCl pH 6.8, 10% SDS, 25% glycerol, 0.1% bromphenol blue) and placed on the gel. After electrophoresis the gels were silver-stained (Wray et al., Anal.Biochem. 118, 1981, 197-203).

The molecular weights of the protein bands were estimated by means of the following reference proteins: myosin (200 kD), beta-galactosidase (116, 3 kD), phosphorylase b (97.4 kD), bovine serum albumin (66.2 kD), ovalbumin (42.7 kD), bovine carbonic anhydrase (31.0 kD), soybean trypsin inhibitor (21.5 kD) and lysozyme (14.4 kD).

## Isoelectric focussing

For isoelectric focussing (IEF) on slab gels a Pharmacia Phast System was used with a ready-for-use 5% PAA gel containing pharmalyte 3-9. The isoelectric point was determined using the following reference proteins: trypsinogen (pI 9.30), lentil lectin alkaline (pI 8.65), lentil lectin mediate (pI 8.45), lentil lectin acid (pI 8.15), horse myoglobin alkaline (pI 7.35), horse myoglobin acid (pI 6.85), human carbonic anhydrase B (pI 6.55), bovin carbonic anhydrase (pI 5.85), beta-lactoglobulin A (pI 5.20), soybean trypsin inhibitor (pI 4.55) and amyloglucosidase (pI 3.50). The gels were automatically stained according to the Phast System with Coomassie brilliant blue.

## Determination of kinetic parameters

The Km and Vmax of the peptidase were determined for the substrate $leu_3$. The hydrolysis of $leu_3$ was determined by measuring the release of leucine in a coupled enzyme reaction in which o-dianisidine (reduced) is oxidized to obtain a brown precipitate. The enzyme reactivities were measured at 30° C, unless otherwise mentioned.

## Protein determination

Protein concentrations were determined by the method of Lowry et al., J.Biol.Chem. 193, 1951, 265-275; by means of bovine serum albumin as standard.

## Determination of the amino acid sequence at the N-terminus of the protein

The amino acid sequence was determined by means of the Edman breakdown method using an Applied Biosystems model 477A pulse liquid sequencer. Samples were prepared by electroblotting on polyvinylidene difluoride (PVDF) membranes according to Matsudaira, J.Biol.Chem.262, 1987, 10035-10038; and after staining with Coomassie blue the amino acid sequence was immediately determined.

## Chemicals

All the chemicals used were commercially available reagent grade substances.

## RESULTS

## Enzyme purification

By means of different methods a tripeptidase of L.lactis subsp. cremoris Wg2 could be purified to homogeneity. A rapid and easy procedure consisted of four successive column chromatography steps which will be elucidated hereinbelow. The resulting activity yields are listed in Table A.

Step a.

After DEAE Sephacel column chromatography of the cell-free extract it turned out that leu$_3$ hydrolysing activity was eluted between 0.18 and 0.24 M NaCl. In this fraction 86.6% of the total activity was recovered in 23.4% of the total amount of protein.

Step b.

The active fraction was injected onto a Phenyl-Sepharose column, and after chromatography the activity was eluted in 2 fractions: the first fraction between 1.6 and 1.4 M NaCl and the second fraction between 55 and 45 mM NaCl.

Contrary to the first fraction, the second fraction also showed lysyl-p-nitroanilide hydrolysis, which proved to be the result of aminopeptidase activity. The first fraction showing leu-gly-gly hydrolysis to leu and gly-gly was selected for a further purification. The specific activity in this fraction was increased 23-fold with respect to the injected sample.

Steps c and d.

For the further purification 2 different gel filtrations were carried out. The concentrated peptidase fraction was placed on a G-100 SF column. After elution the specific activity of the leu$_3$ hydrolysing fraction was increased 2.5-fold. This fraction was placed on a HPLC TSK G3000 SW column. This second gel filtration gave a fraction with leu$_3$ hydrolysing activity showing one single protein band with SDS-PAGE after silver staining. In total, 19% of the initial enzyme activity was recovered, and the specific activity was increased 378-fold.

Molecular weight and isoelectric point

The molecular weight of the peptidase was estimated at 105000 (with SDS-PAGE) and at 120000 (with HPLC on a TSK G3000 SW column against the same reference proteins). With SDS-PAGE in the presence of $\beta$-mercaptoethanol one single protein band was likewise found at 105000, which indicates that the enzyme does not consist of several subunits linked by disulphide bridges.

The pI of the enzyme was estimated at 3.7.

Temperature dependence and effect of the pH on the enzyme activity

The effect of the temperature on the peptidase activity was determined by measuring the hydrolysis of leu$_3$ within the range of from 15 to 80° C.

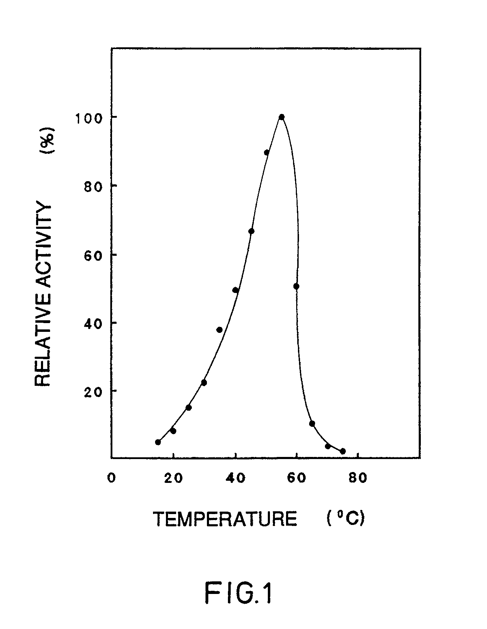

The temperature optimum of the peptidase for leu$_3$ hydrolysis was at 55° C (Fig. 1). At temperatures below 20° C and above 65° C the leu$_3$ hydrolysis activity was less than 10%. After 20 min preincubation at 55° C the enzyme activity was inhibited by 52%. The effect of the pH on the enzyme activity was examined within the range of from 4 to 12 by means of a poly buffer composed of 20 mM of each of the substances malic acid, 2-(N-morpholino)ethanesulphonic acid (MES), HEPES, boric acid and ethylammonium chloride and adjusted at the appropriate pH. The enzyme was equilibrated for 10 min in the polybuffer before leu$_3$ was added (final concentration 2 mM) The optimum activity of the peptidase was found at pH 7.5. At pH values below 4.5 and above 11.5 the leu$_3$ hydrolysis activity had disappeared completely.

Substrate specificity

The hydrolytic effect of the enzyme on several peptides is summarised in Table B. The hydrolysis products were analysed by thin-layer chromatography (TLC). It was found that the purified peptidase could hydrolyse all the tested tripeptides, except those with proline as the second amino acid. Only the N-terminal amino acid of the tripeptides was seperated, so that a free amino acid and a dipeptide were formed. Dipeptides, tetrapeptides or oligopeptides did not prove to be hydrolysed.

Kinetic parameters

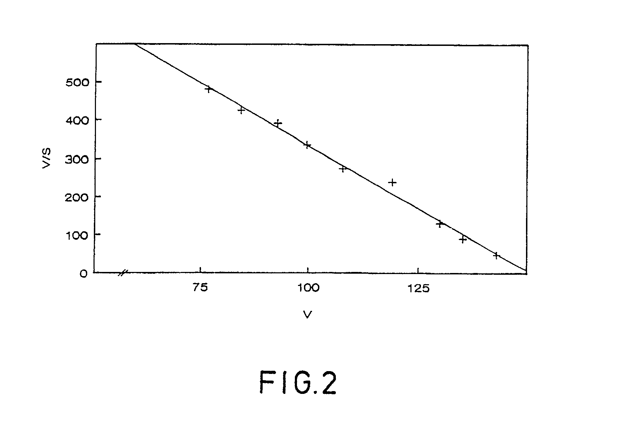

The kinetic parameters of the peptidase were determined for leu$_3$ as substrate, and the Km and Vmax values for leu$_3$ proved to be respectively 0.15 mM and 151 $\mu$mol/min.mg protein (see Fig. 2).

Effect of inhibitors on the peptidase activity

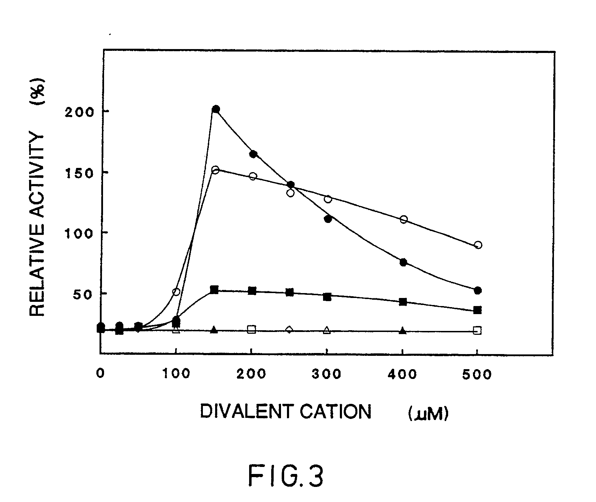

Incubation of the peptidase (0.42 $\mu$g protein/ml) with the divalent cations-chelating agent EDTA (0.2 mM) led to inhibition of the enzyme activity by 79%. After removal of excess EDTA by dialysis against 20 mM HEPES (pH 7.0 at 4°C, 20 hours) the activity could be maximally restored with 150 $\mu$M $Zn^{2+}$, $Mn^{2+}$ and $Co^{2+}$ to respectively 202, 152 and 50% of the original activity (see Fig. 3). $Zn^{2+}$ and $Mn^{2+}$ proved to be inhibitory at concentrations above 150 $\mu$M. $Fe^{2+}$, $Ca^{2+}$, $Mg^{2+}$ and $Cu^{2+}$ were not able to reactivate the enzyme. All the metal ions were added in the form of chlorides to exclude a possible effect of the anions.

Incubation of the peptidase (0.42 $\mu$g protein/ml), not treated with EDTA, with 125 $\mu$M $Zn^{2+}$ or $Co^{2+}$ resulted in a stimulation of the peptidase activity to 175%. $Cu^{2+}$ inhibited the activity, and at concentrations above 125 $\mu$M the enzyme was inactivated completely. $Mn^{2+}$, $Ca^{2+}$, $Fe^{2+}$ had no effect on the activity. The sulphydryl group reagents p-chloromercuribenzoate (pCMB), p-chloromercuribenzenesulphonate (pCMBS) and o-(3-hydroxymercuri-2-methoxypropyl)carbamylphenoxyacetate (mersalyl) (0.2 mM) stimulated the untreated enzyme activity to about 200% (Table C). N-ethylmaleimide (NEM; 0.5 mM) slightly inhibited the activity, whereas cystine (0.05 mM) and the dithiol reagent phenylarsine oxide (0.1 mM) had no effect on the enzyme activity. The reducing agents DTT (1 mM) and $\beta$-mercaptoethanol (0.4 mM) gave a strong inhibition of the enzyme activity, and this inhibition could be partly removed with the oxidising reagent ferricyanide $(Fe[CN]_6)^{3-}$ (3 mM).

Discussion of the Figures

Fig. 1. Effect of the temperature on the activity of the purified peptidase. The relative activity was determined by incubation of the enzyme at different temperatures and measuring $leu_3$ hydrolysis.

Fig. 2. Eadie-Hofstee plot for $leu_3$ hydrolysis through the peptidase. The hydrolysis of $leu_3$ was determined as already described.

Fig. 3. Effect of divalent cations on the peptidase activity. Reaction mixtures containing peptidase inactivated by EDTA (0.42 $\mu$g protein/ml) were incubated with the divalent cations for 10 min at pH 7 at 20°C before the substrate $leu_3$ was added.

Symbols: ( ● ) $Zn^{2+}$, (O) $Mn^{2+}$, ( ■ ) $Co^{2+}$, ( □ ) $Fe^{2+}$, ( Δ ) $Ca^{2+}$, ( ▲ ) $Mg^{2+}$, ( ◊ ) $Cu^{2+}$.

## TABLE A

Purification of a leu$_3$ hydrolysing peptidase from L.lactis subsp. cremoris Wg2

| Purification step | Total protein (mg) | Total activity (µmol/min) | Yield (%) | Specific activity (µmol/mg.min) | Degree of purification (number of times) |
|---|---|---|---|---|---|
| cell-free extract | 725.2 | 372.7 | 100 | 0.51 | 1 |
| DEAE-Sephacel | 169.4 | 322.6 | 86.6 | 1.90 | 3.7 |
| Phenyl-Seph. | 4.77 | 208.1 | 55.8 | 43.6 | 85.5 |
| G-100 SF | 0.99 | 105.5 | 28.3 | 107.1 | 210 |
| TSK G3000 SW | 0.37 | 70.5 | 18.9 | 192.7 | 378 |

EP 0 440 303 A1

EP 0 440 303 A1

TABLE B

Substrate specificity of a peptidase from L.lactis subsp. cremoris Wg2.
Peptide hydrolysis was analyzed by TLC.

| Substrate | activity | Substrate | activity |
|---|---|---|---|
| Leu-Leu-Leu | + | Leu-Leu | − |
| Ala-Val-Leu | + | Phe-Leu | − |
| Gly-Phe-Leu | + | Leu-Met | − |
| Gly-Leu-Tyr | + | Leu-Gly | − |
| Gly-Ser-Ala | + | Gly-Gly | − |
| Gly-Tyr-Ala | + | Glu-Glu | − |
| Gly-Pro-Ala | − | Glu-Lys | − |
| Gly-His-Gly | + | Glu-Val | − |
| Gly-Gly-Leu | + | Glu-Ala | − |
| Gly-Gly-Phe | + | Ala-Asp | − |
| Gly-Gly-Gly | + | | |
| Arg-Gly-Gly | + | $Ala_2$ | − |
| His-Gly-Gly | + | $Ala_3$ | + |
| Leu-Gly-Gly | + | $Ala_4$ | − |
| Met-Gly-Gly | + | $Ala_5$ | − |
| Phe-Gly-Gly | + | $Ala_6$ | − |
| Pro-Gly-Gly | + | | |
| Ser-Gly-Gly | + | Gly-Pro-Gly-Gly | − |
| Trp-Gly-Gly | + | Bradykinin | − |
| Tyr-Gly-Gly | + | Met-enkephalin | − |
| Val-Gly-Gly | + | Neurotensin | − |
| Ala-His-Ala | + | Substance P | − |
| Ala-Pro-Ala | − | oxidized $\beta$-chain | − |
| Ala-Pro-Gly | − | insulin | |
| Ala-Pro-Phe | − | | |
| Val-Pro-Leu | − | | |
| Orn-Orn-Orn | + | | |
| Glu-Val-Phe | + | | |

+ : hydrolysis
− : no hydrolysis

## TABLE C

Effects of chemical agents on the peptidase activity. Reaction mixtures containing 0.84 $\mu$g enzyme/ml were incubated with the chemical agent for 10 min at pH 7 (20°C) before the substrate leu$_3$ was added.

*: after 10 min incubation with 1 mM DTT the enzyme was incubated with 3 mM ferricyanide for 10 min.

| Reagent (mM) | relative activity (%) |
|---|---|
| none | 100 |
| pCMB (0.2) | 191 |
| pCMBS (0.2) | 205 |
| Mersalyl (0.2) | 188 |
| NEM (0.5) | 76 |
| Phenylarsine oxide (0.1) | 118 |
| Cystine (0.05) | 118 |
| DTT (1) | 4 |
| $\beta$-Mercaptoethanol (0.4) | 14 |
| DTT (1) + $(Fe[CN]_6)^{3-}$ (3) * | 64 |

## Claims

1. Tripeptidase of lactic acid bacteria in isolated form, characterized by a molecular weight of 105 kD ± 5 kD; an isoelectric point of 3.7 ± 0.4; a substrate range comprising only tripeptides, with the exception of those with proline as the second amino acid, and comprising no dipeptides, no oligopeptides such as ala$_4$, ala$_5$, ala$_6$, and no polypeptides; a hydrolysis activity with a temperature optimum at 55°C ± 5°C and a pH optimum at 7.5 ± 0.5; and sensitivity to both the metal chelating agent EDTA and the reducing agents dithiothreitol and $\beta$-mercaptoethanol.

2. Tripeptidase according to claim 1, originating from lactic acid lactococci.

3. Tripeptidase according to claim 2, originating from the lactic acid lactococcus Lactococcus lactis.

4. Tripeptidase according to claim 2, originating from the lactic acid lactococcus Lactococcus lactis subsp. cremoris.

5. Tripeptidase according to claim 2, originating from the lactic acid lactococcus Lactococcus lactis subsp. cremoris Wg2.

6. Tripeptidase according to any of claims 1-5, obtained by isolation thereof from a cell mass, a culture medium or a separation product of lactic acid bacteria in which the tripeptidase is naturally expressed, or from a cell mass, a culture medium or a separation product of an organism which, by genetic engineering, has acquired the ability to express the tripeptidase.

7. Organisms, in particular microorganisms, which, by genetic engineering, have acquired either the ability foreign to them to express a tripeptidase according to any of claims 1-6 or the ability of a stronger expression of a tripeptidase according to any of claims 1-6 than they naturally possess.

8. A recombinant polynucleotide, in particular a recombinant DNA, comprising the genetic information coding for a tripeptidase according to any of claims 1-6.

9. A process for preparing a tripeptidase according to any of claims 1-6, characterized in that a cell-free extract of an organism, in particular a microorganism, in which the tripeptidase is expressed, is subjected to one or more ion exchange column chromatography and gel filtration steps, in which of each eluate collected in fractions one or more of the fractions with hydrolysis activity with respect to $leu_3$ and/or leu-gly-gly are selected.

10. The use of a tripeptidase according to any of claims 1-6 for preparing polyclonal or monoclonal antibodies having affinity and/or specificity for the tripeptidase.

11. Polyclonal and monoclonal antibodies having affinity and/or specificity for a tripeptidase according to any of claims 1-6.

12. The use of polyclonal or monoclonal antibodies according to claim 11 for detecting, removing and/or isolating the tripeptidase.

13. The use of a recombinant polynucleotide according to claim 8 as a transformation marker, in which the expression of the tripeptidase for which the polynucleotide contains the genetic information is detected by means of an appropriate substrate of the tripeptidase or by means of polyclonal or monoclonal antibodies having affinity and/or specificity for the tripeptidase.

14. A process for preparing foods such as cheese, whippable products and meat products, characterized by using a tripeptidase according to any of claims 1-6 and/or an organism according to claim 7.

15. Foods such as cheese, whippable products and meat products, obtained using a tripeptidase according to any of claims 1-6 and/or an organism according to claim 7.

FIG.1

FIG.2

14

FIG.3

European Patent
Office

EUROPEAN SEARCH REPORT

Application Number

EP    91 20 0189
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF DAIRY SCIENCE. vol. 67, no. 11, 1984, CHAPAIGN, ILLINOIS U pages 2483 - 2492; S.KAMINOGAWA et al.: "Aminopeptidase profiles of lactic Streptococci" * the whole document * | 1-4, 6 | C12N9/48 C12N15/57 A23C19/032 A23C13/16 A23L1/31 C12P21/08 G01N33/68 |
| A | JOURNAL of APPLIED BACTERIOLOGY. vol. 58, 1985, LONDON,GB. pages 449 - 456; J.KOLSTAD et al: "Comparative peptide specificity of cell wall,membrane and intracellular peptidases of group N Streptococci." * the whole document * | 1-4, 6 | |
| A | JOURNAL OF DAIRY RESEARCH . vol. 42, 1975, LONDON, GB. pages 147 - 155; L.MOU et al: "Peptidase activities in group N Streptococci." * the whole document * | 1-4, 6 | |
| A | JOURNAL OF APPLIED BACTERIOLOGY vol. 47, 1979, LONDON, GB. pages 65 - 73; A.J. CLIFFE et al: "An electrophoretic study of peptidases in starter streptococci and in cheddar cheese." * the whole document * | 1-4, 6 | **TECHNICAL FIELDS SEARCHED (Int. Cl.5)**<br><br>C12N |
| D,A | JOURNAL OF APPLIED BACTERIOLOGY. vol. 46, 1979, LONDON; GB. pages 455 - 463; B. A. LAW: "Extracellular Peptidases in group N Streptococci used as Cheese Starters." * the whole document * | 1-4, 6 | |

-/--

The present search report has been drawn up for all claims

| Place of search | Date of completion of the search | Examiner |
|---|---|---|
| THE HAGUE | 22 APRIL 1991 | LE CORNEC N.D.R. |

EPO FORM 1503 03.82 (P0401)

European Patent

Office

**EUROPEAN SEARCH REPORT**

Application Number

EP    91 20 0189

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | APPLIED AND ENVIRONMENTAL MICROBIOLOGY. vol. 56, no. 6, June 1990, WASHINGTON,D.C. USA . pages 1839 - 1843; B.W. BOSMAN et al: "Purification and Characterization of a Tripeptidase from Lactococcus lactis subsq. cremoris Wg2." * the whole document * | 1-6, 9 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 22 APRIL 1991 | LE CORNEC N.D.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)